(19) Europäisches Patentamt
European Patent Office
Office européen des brevets

(11) Veröffentlichungsnummer : **0 580 554 A1**

(12) **EUROPÄISCHE PATENTANMELDUNG**

(21) Anmeldenummer : **93810507.9**

(51) Int. Cl.$^5$ : **C07D 213/74**, C09B 33/12

(22) Anmeldetag : **14.07.93**

(30) Priorität : **23.07.92 CH 2351/92**

(43) Veröffentlichungstag der Anmeldung :
**26.01.94 Patentblatt 94/04**

(84) Benannte Vertragsstaaten :
**BE CH DE ES FR GB IT LI PT**

(71) Anmelder : **CIBA-GEIGY AG**
**Klybeckstrasse 141**
**CH-4002 Basel (CH)**

(72) Erfinder : **Herzig, Paul**
**Ackerstrasse 52**
**CH-4057 Basel (CH)**
Erfinder : **Andreoli, Anton**
**Im Hirshalm 58**
**CH-4125 Riehen (CH)**

(54) **Mischungen von Aminopyridinverbindungen, Verfahren zu deren Herstellung und deren Verwendung.**

(57) Die Erfindung betrifft Mischungen von Aminopyridinverbindungen, welche eine Verbindung der Formel

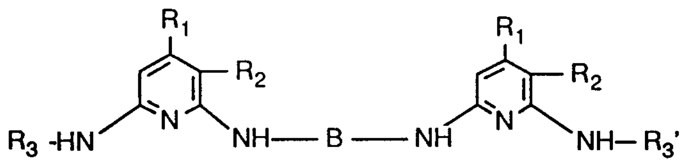

(1a)

zusammen mit mindestens einer Verbindung der Formeln

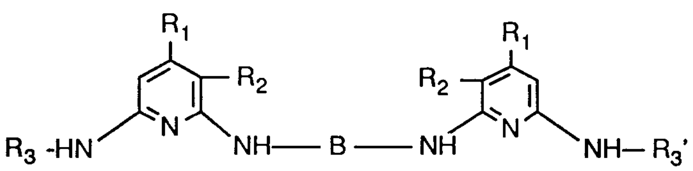

(1b)

und

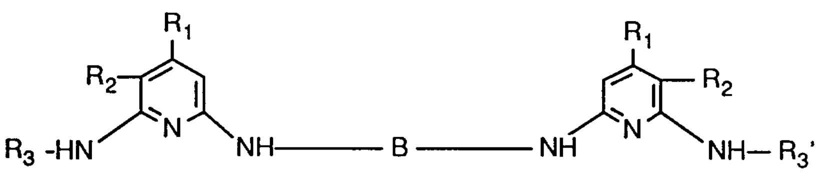

(1c)

enthalten, wobei $R_1$, $R_2$, $R_3$, $R_3$' und B die in Anspruch 1 angegebenen Bedeutungen haben.
Die erfindungsgemässen Mischungen eignen sich zur Herstellung von Farbstoffen.

EP 0 580 554 A1

Die vorliegende Erfindung betrifft neue Mischungen von Aminopyridinverbindungen, Verfahren zu deren Herstellung und deren Verwendung als Zwischenprodukte zur Herstellung von Farbstoffen.

Gegenstand der vorliegenden Erfindung sind Mischungen von Aminopyridinverbindungen, welche dadurch gekennzeichnet sind, dass sie eine Verbindung der Formel

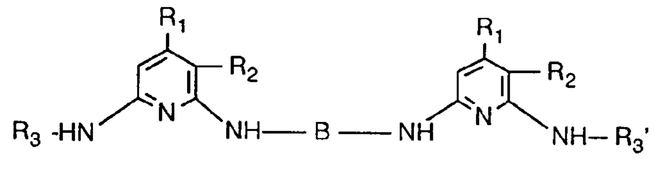 (1a)

zusammen mit mindestens einer Verbindung der Formeln

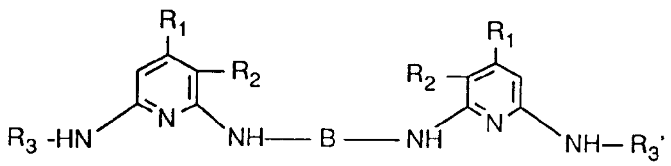 (1b)

und

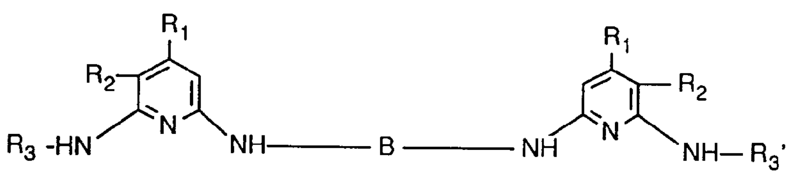 (1c)

enthalten, wobei
$R_1$ $C_1$-$C_4$-Alkyl,
$R_2$ Cyano, Carbamoyl oder Sulfomethyl ist,
$R_3$ und $R_3$' unabhängig voneinander Wasserstoff oder gegebenenfalls substituiertes und mit Ausnahme von Methyl gegebenenfalls durch Sauerstoff unterbrochenes $C_1$-$C_{12}$-Alkyl bedeuten und
B gegebenenfalls substituiertes und gegebenenfalls durch Sauerstoff unterbrochenes $C_2$-$C_{12}$-Alkylen ist.

Als $C_1$-$C_4$-Alkyl kommen für $R_1$ Methyl, Aethyl, Propyl, Isopropyl, Butyl, Isobutyl, sek.-Butyl oder tert.-Butyl, insbesondere Methyl, in Betracht.

Für $R_2$ ist die Bedeutung als Cyano oder Carbamoyl bevorzugt.

Substituenten der Reste $R_3$ und $R_3$' als $C_1$-$C_{12}$-Alkyl sind z.B. Hydroxyl, Sulfo, Sulfato, sowie gegebenenfalls weitersubstituiertes Phenyl oder Naphthyl, wobei die Phenyl- und Naphthylreste insbesondere gegebenenfalls durch $C_1$-$C_4$-Alkyl, $C_1$-$C_4$-Alkoxy, $C_2$-$C_4$-Alkanoylamino, Halogen oder Sulfo weitersubstituiert sind.

Die Reste B, $R_3$ und $R_3$' können durch Sauerstoff unterbrochen sein, wie z.B. durch 1 bis 3, insbesondere 1 oder 2 Reste -O-.

Bevorzugt sind $R_3$ und $R_3$' unabhängig voneinander Wasserstoff oder gegebenenfalls durch Hydroxyl, Sulfo, Sulfato oder gegebenenfalls weitersubstituiertes Phenyl oder Naphthyl substituiertes und mit Ausnahme von Methyl gegebenenfalls durch Sauerstoff unterbrochenes $C_1$-$C_{12}$-Alkyl. Die Phenyl- und Naphthylreste sind vorzugsweise gegebenenfalls durch $C_1$-$C_4$-Alkyl, $C_1$-$C_4$-Alkoxy, $C_2$-$C_4$-Alkanoylamino, Halogen oder Sulfo weitersubstituiert.

Besonders bevorzugt sind $R_3$ und $R_3$' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes $C_1$-$C_{12}$-Alkyl.

Ganz besonders bevorzugt sind $R_3$ und $R_3$' unabhängig voneinander Wasserstoff, gegebenenfalls durch Hydroxyl, Sulfo oder Sulfato substituiertes und mit Ausnahme von Methyl gegebenenfalls durch 1 bis 3, insbesondere 1 oder 2 Reste -O- unterbrochenes $C_1$-$C_8$-Alkyl.

Als Beispiele für Reste $R_3$ und $R_3$' seien Methyl, Aethyl, Propyl, Isopropyl, Butyl, Isobutyl, sek.-Butyl, tert.-Butyl oder geradkettiges oder verzweigtes Pentyl, Hexyl oder Octyl, 2-Hydroxyäthyl, 3-Hydroxypropyl, 2-Sulfatoäthyl 3-Sulfatopropyl, 2-Sulfoäthyl, 3-Sulfopropyl, 2-Methoxyäthyl, 3-Methoxypropyl, sowie Reste der

Formeln $-(CH_2)_2-O-(CH_2)_2-OH$ und $-(CH_2)_2-O-(CH_2)_2-OSO_3H$ genannt.

Die Reste $R_3$ und $R_3$' haben vorzugsweise identische Bedeutungen.

Der Rest B ist bevorzugt gegebenenfalls durch Hydroxyl, Sulfo oder Sulfato substituiertes und gegebenenfalls durch Sauerstoff unterbrochenes $C_2$-$C1_2$-Alkylen.

Besonders bevorzugt handelt es sich bei dem Rest B um $C_2$-$C_8$-Alkylen, welches gegebenenfalls durch Hydroxyl, Sulfo oder Sulfato substituiert ist und gegebenenfalls durch 1 bis 3, insbesondere 1 oder 2 Reste -O- unterbrochen ist.

Ganz besonders bevorzugt handelt es sich bei dem Rest B um $C_2$-$C_6$-Alkylen, insbesondere um 1,2-Aethylen, 1,3-Propylen oder 1,6-Hexylen.

Als Beispiele für den Rest B seien 1,2-Aethylen, 1,3-Propylen, 1,4-Butylen, 1,5-Pentylen, 1,6-Hexylen sowie Reste der Formeln $-(CH_2)_3-O-(CH_2)_4-O-(CH_2)_3-$, $-(CH_2)_2-O-(CH_2)_2$,

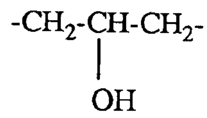 und 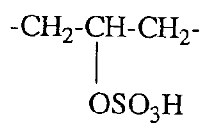

genannt.

Bevorzugt sind Mischungen von Aminopyridinverbindungen, worin $R_1$ Methyl, $R_2$ Cyano oder Carbamoyl ist, $R_3$ und $R_3$' identische Bedeutungen haben und Wasserstoff oder gegebenenfalls durch Hydroxyl, Sulfo oder Sulfato substituiertes und mit Ausnahme von Methyl gegebenenfalls durch Sauerstoff unterbrochenes $C_1$-$C_{12}$-Alkyl sind und B gegebenenfalls durch Hydroxyl, Sulfo oder Sulfato substituiertes und gegebenenfalls durch Sauerstoff unterbrochenes $C_2$-$C_{12}$-Alkylen ist.

Besonders bevorzugt sind Mischungen von Aminopyridinverbindungen, worin $R_1$ Methyl, $R_2$ Cyano oder Carbamoyl ist, $R_3$ und $R_3$' identische Bedeutungen haben und Wasserstoff, gegebenenfalls durch Hydroxyl, Sulfo oder Sulfato substituiertes und mit Ausnahme von Methyl gegebenenfalls durch 1 bis 3, insbesondere 1 oder 2 Reste -O- unterbrochenes $C_1$-$C_8$-Alkyl sind und B $C_2$-$C_8$-Alkylen ist, welches gegebenenfalls durch Hydroxyl, Sulfo oder Sulfato substituiert ist und gegebenenfalls durch 1 bis 3, insbesondere 1 oder 2 Reste -O- unterbrochen ist.

Als Mischungen sind insbesondere solche von Interesse, welche eine Verbindung der Formel (1a) zusammen mit einer Verbindung der Formel (1b) enthalten.

Ein weiterer Gegenstand der vorliegenden Erfindung ist ein Verfahren zur Herstellung der erfindungsgemässen Mischungen von Aminopyridinverbindungen, welches dadurch gekennzeichnet ist, dass man eine Verbindung der Formel

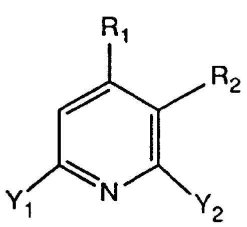

(2),

worin $R_1$ und $R_2$ die oben angegebenen Bedeutungen haben und $Y_1$ und $Y_2$ je eine Abgangsgruppe sind, mit Aminen der Formeln

$$NR_2-R_4 \ (3) \text{ und } NH_2-B-NH_2 \qquad (4),$$

worin B die oben angegebenen Bedeutungen hat und $R_4$ die für $R_3$ und $R_3$' angegebenen Bedeutungen hat, umsetzt und gegebenenfalls eine weitere Umwandlungsreaktion anschliesst.

Als Abgangsgruppe $Y_1$ und $Y_2$ kommt insbesondere Chlor in Betracht.

Für $R_1$, $R_2$ und B gelten die oben genannten Bedeutungen und Bevorzugungen. Für $R_4$ gelten die oben für $R_3$ und $R_3$' angegebenen Bedeutungen und Bevorzugungen.

Die Umsetzung erfolgt beispielsweise bei einer Temperatur von ca. 70 bis 130°C, insbesondere 70 bis 90°C, in einem Lösungsmittel, wie z.B. N,N-Diäthylanilin, Triäthylamin, höheren Alkoholen oder Ketonen, oder man verwendet die Amine der Formeln (3) und (4) selbst als Lösungsmittel. Die Umsetzung erfolgt in Gegenwart einer Base, wobei ein geeignetes Lösungsmittel, wie z.B. Triäthylamin, selbst als Base fungieren kann oder eine Base, wie z.B. Natriumcarbonat oder Kaliumcarbonat, zugesetzt wird.

Durch geeignete Wahl der Reaktionsbedingungen können die Abgangsgruppen $Y_1$ und $Y_2$ der Verbindung

der Formel (2) stufenweise substituiert werden, was die Isolierung der einzelnen Mono- oder Disubstitutionsprodukte erlaubt.

So wird in einem ersten Schritt bei niedrigerer Temperatur, wie z.B. 10 bis 50°C, vorwiegend der Rest $Y_1$ abgespalten, während in einem zweiten Schritt bei höherer Temperatur, wie z.B. 70 bis 120°C, der Rest $Y_2$ abgespalten wird.

Werden somit die Amine der Formeln (3) und (4) nicht zusammen, sondern nacheinander zugegeben, so kann entweder die Verbindung der Formel (1b) oder die Verbindung der Formel (Ic) als Hauptprodukt erhalten werden. Hierbei hat es sich als vorteilhaft erwiesen, das nach Reaktion des im ersten Schritt bei niedrigerer Temperatur erhaltene monosubstituierte Zwischenprodukt zu isolieren und danach den zweiten Schritt, die Umsetzung des zweiten Amins bei höherer Temperatur, auszuführen. Die Reaktion kann jedoch prinzipiell ohne Zwischenisolierung ausgeführt werden.

Wird daher im ersten Schritt bei niedriger Temperatur das Amin der Formel (3) und im zweiten Schritt bei höherer Temperatur das Amin der Formel (4) zugegeben, so wird als Hauptverbindung die Verbindung der Formel (1b) erhalten. Werden die Amine in umgekehrter Reihenfolge eingesetzt, so wird als Hauptverbindung die Verbindung der Formel (1c) erhalten.

Die Verbindung der Formel (1a) ist in der Regel unabhängig von der Reihenfolge der Zugabe der Amine der Formeln (3) und (4) ein Bestandteil der erhaltenen Mischung.

Gegebenenfalls kann im Anschluss an die Umsetzung der Verbindung der Formel (2) mit den Aminen der Formeln (3) und (4) eine weitere Umwandlungsreaktion angeschlossen werden. So kann im Anschluss an die Reaktion der Rest $R_2$ in der Bedeutung als Cyano in die Carbamoylgruppe überführt werden. Diese Reaktion erfolgt in der Regel in konzentrierter Schwefelsäure bei erhöhter Temperatur.

Die Verbindungen der Formeln (2), (3) und (4) sind bekannt oder können in Analogie zu bekannten Verbindungen hergestellt werden.

Die erfindungsgemässen Mischungen eignen sich als Zwischenprodukte für die Herstellung von Farbstoffen. Die erfindungsgemässen Mischungen können beispielsweise als Zwischenprodukte, wie Kupplungskomponenten, zur Herstellung von Azofarbstoffen eingesetzt werden. Besondere Eignung finden die erfindungsgemässen Mischungen bei der Herstellung von Azofarbstoffen, insbesondere reaktiven Azofarbstoffen. Die mit den erfindungsgemässen Mischungen erhältlichen Farbstoffe eignen sich zum Färben oder Bedrucken von stickstoffhaltigen oder hydroxylgruppenhaltigen Fasermaterialien. Die Herstellung der Farbstoffe erfolgt nach an sich bekannten Methoden.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anders vermerkt. Gewichtsteile stehen zu Volumenteilen im Verhältnis von Kilogramm zu Liter.

Beispiel 1:

187 Teile 2,6-Dichlor-3-cyan-4-methylpyridin werden bei einer Temperatur von 20 bis 30°C in 427 Teile Aethanolamin eingetragen. Man rührt das Gemisch 2 Stunden bei 20 bis 30°C. Die klare braune Lösung wird auf 3000 Teile Eiswasser ausgetragen. Der entstandene Niederschlag wird abfiltriert, mit Wasser gewaschen und getrocknet.

Man erhält 180 Teile eines Gemisches der Verbindungen der Formeln

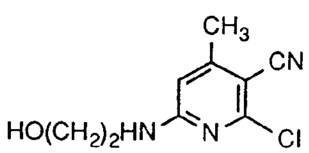 (101) und 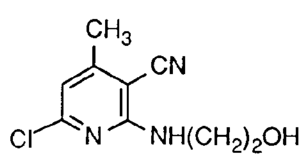 (102),

wobei das Verhältnis der Verbindung der Formel (101) zur Verbindung der Formel (102) 3:1 ist.

180 Teile des wie angegeben erhaltenen Gemisches der Verbindungen der Formeln (101) und (102) werden in 685 Teilen Triäthylamin eingetragen und auf Rückfluss erhitzt. Man tropft 31,5 Teile 1,3-Diaminopropan langsam zu und verrührt weitere 5 Stunden am Rückfluss. Nach beendetem Umsatz streut man 45 Teile Natriumcarbonat ein und destilliert das Triäthylamin ab. Der entstandene Rückstand wird auf 3000 Teile Eiswasser ausgetragen und der entstandene Niederschlag abfiltriert und mit Wasser gewaschen. Nach dem Trocknen erhält man 160 Teile eines Gemisches der Verbindungen der Formeln

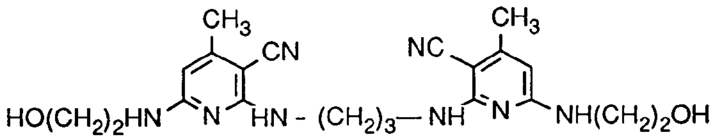

(103),

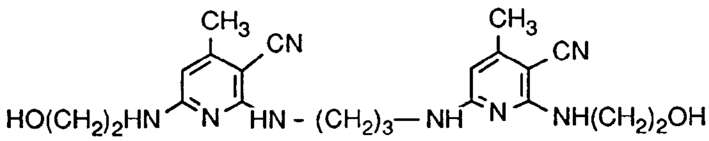

(104)

und

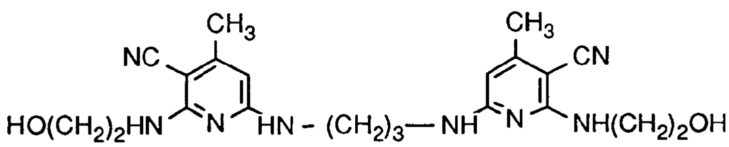

(105),

wobei der Anteil der Verbindung der Formel (103) ca. 60%, der der Verbindung der Formel (104) ca. 35% und der der Verbindung der Formel (105) ca. 5% ist.

Beispiel 2:

187 Teile 2,6-Dichlor-3-cyan-4-methyl-pyridin werden bei einer Temperatur von 40 bis 50°C in 800 Teile Triethylamin eingetragen. Zu dieser Suspension lässt man 37 Teile 1,3-Diaminopropan zutropfen und lässt noch 5 Stunden bei 40 bis 50°C nachrühren. Man erhält eine Suspension, welche die Verbindungen der Formeln

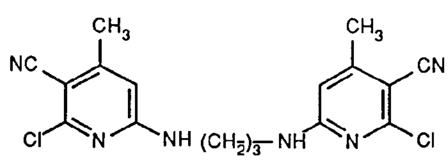

(106),

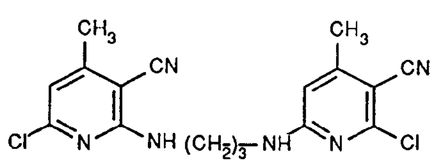

(107)

und

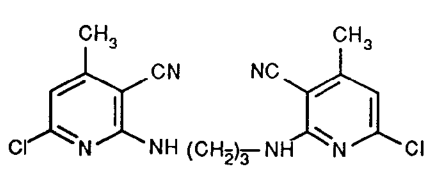

(108)

enthält, wobei der Anteil der Verbindung der Formel (106) ca. 60%, der der Verbindung der Formel (107) ca.

35% und der der Verbindung der Formel (108) ca. 5% ist.

Zu der so erhaltenen Suspension werden 500 Teile Ethanolamin zugegeben. Die Temperatur wird danach auf Rückfluss erhöht und Triethylamin abdestilliert. Während des Abdestillierens des Triethylamins werden nochmals 500 Teile Ethanolamin zugegeben, damit die Suspension nicht zu dick wird. Nach Erreichen einer inneren Temperatur von 115°C wird noch 2 Stunden bei dieser Temperatur nachgerührt. Die dunkle Lösung wird auf Raumtemperatur abgekühlt und auf 6000 Teile Wasser ausgetragen. Die entstandene Suspension wird abfiltriert und mit Wasser gewaschen. Nach dem Trocknen erhält man ca. 150 Teile eines Gemisches der Verbindungen der Formeln

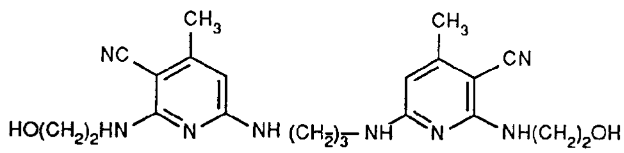

(109),

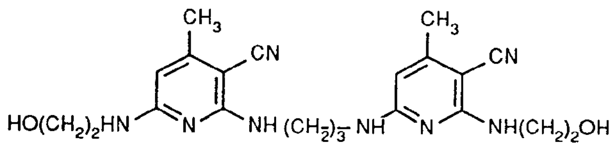

(110)

und

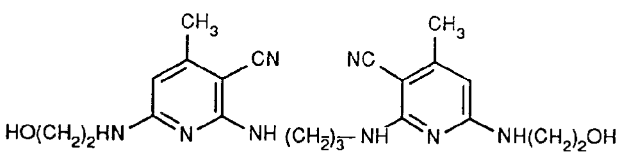

(111),

wobei der Anteil der Verbindung der Formel (109) ca. 60%, der der Verbindung der Formel (110) ca. 35% und der der Verbindung der Formel (111) ca. 5% ist.

Beispiele 3 bis 45:

Analog zu Beispiel 1 können entsprechende Mischungen von Aminopyridinverbindungen erhalten werden, die die in der folgenden Tabelle in Spalte 2 angegebenen Aminopyridinverbindungen als Haupkomponente enthalten. Die Mischungen enthalten drei Komponenten, welche sich nur dadurch unterscheiden, dass die Cyano- bzw. Carbamoylgruppe in 3- oder in 5-Stellung an den jeweiligen Pyridinring gebunden ist.

Tabelle

| Bsp. | Aminopyridinverbindung |
|---|---|
| 3 | 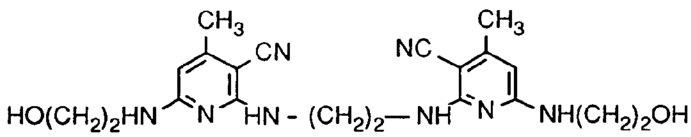 |
| 4 | 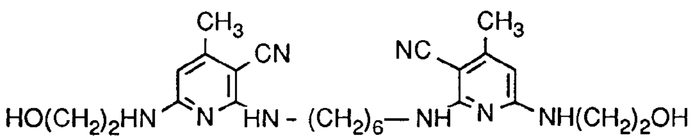 |
| 5 | 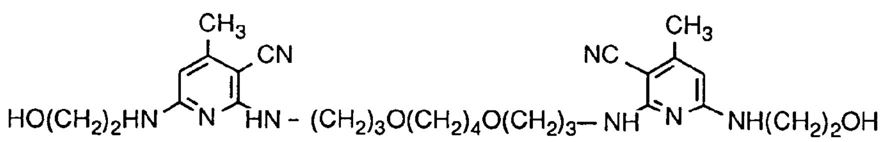 |
| 6 | 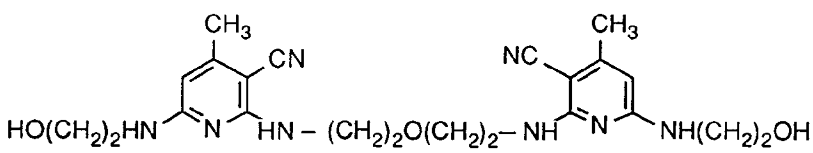 |

7

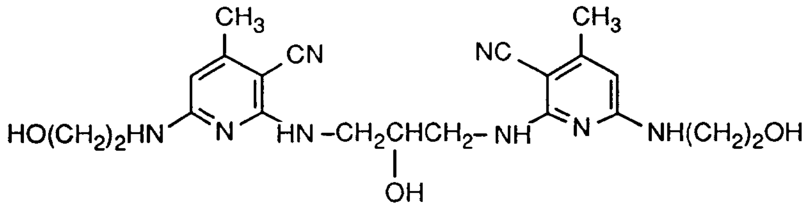

8

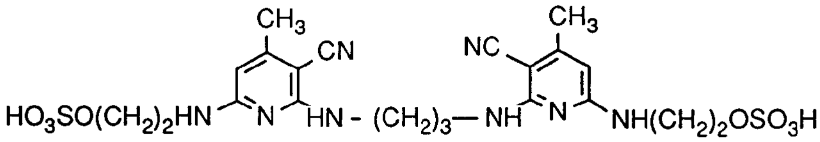

9

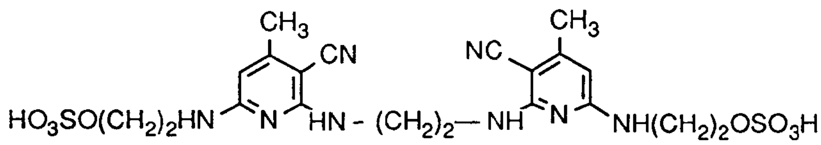

10

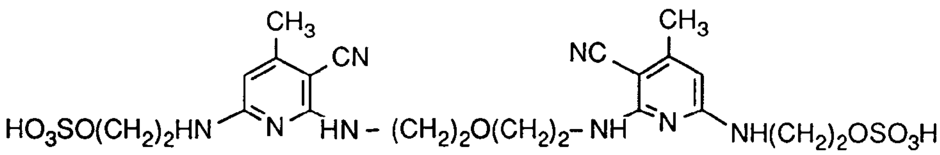

11

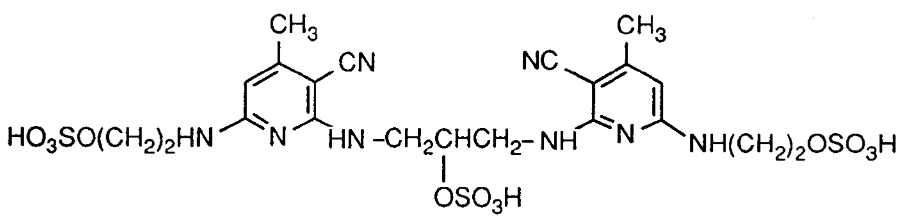

12

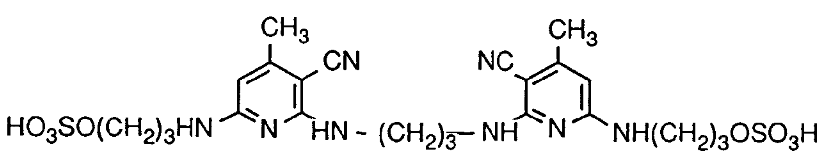

13

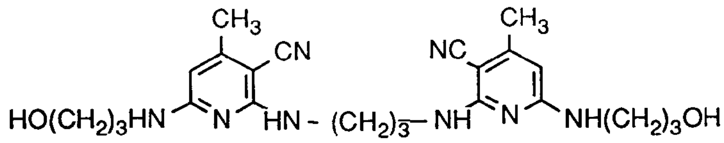

14

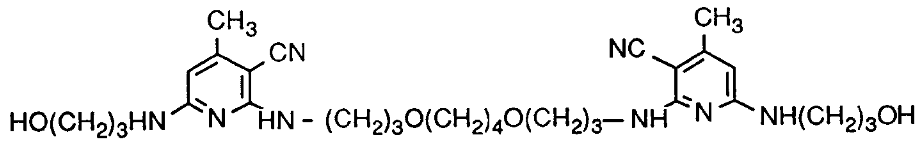

15

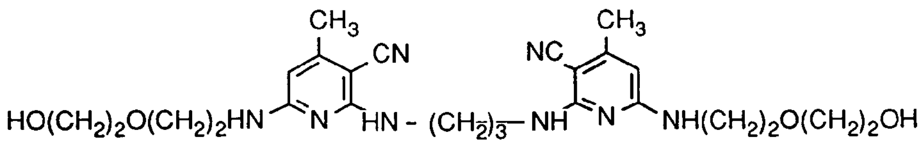

16

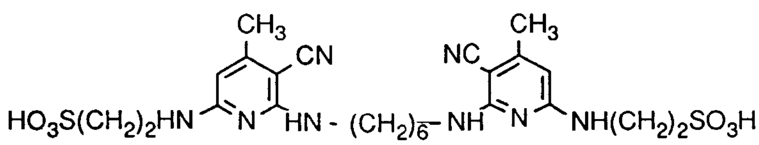

17

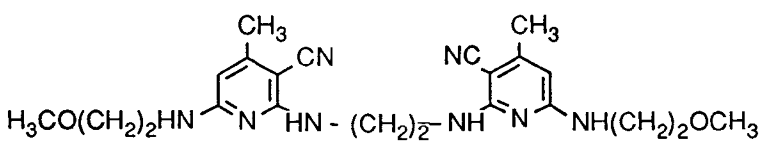

18

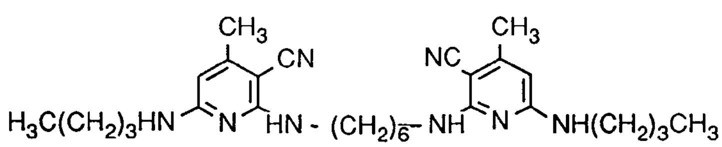

19

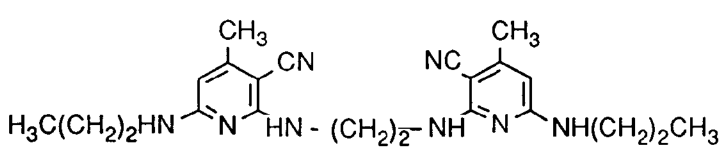

20 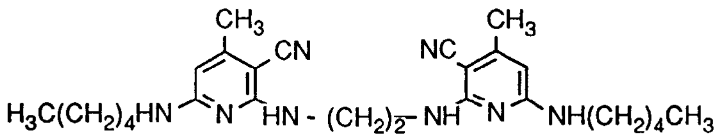

21 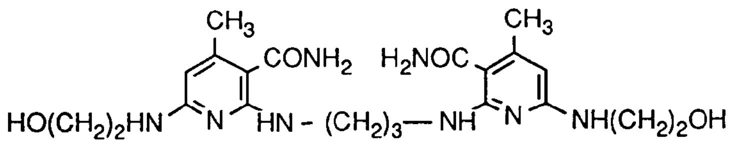

22 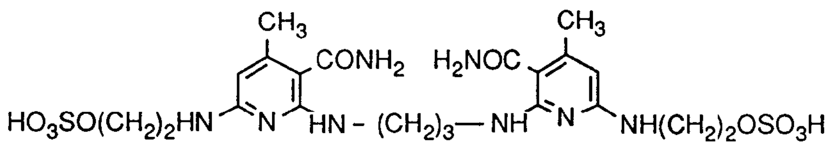

23 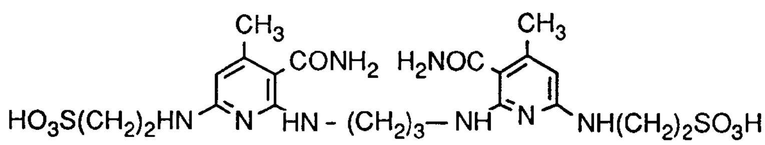

24 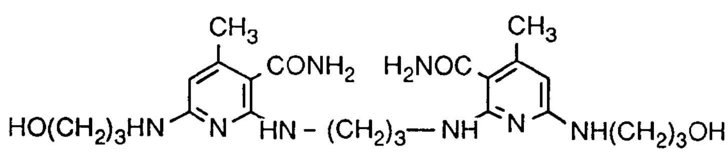

25 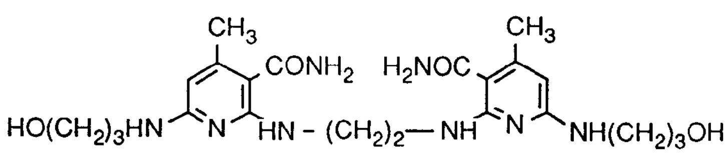

26

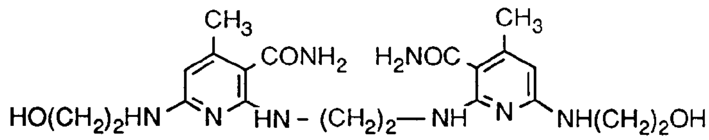

27

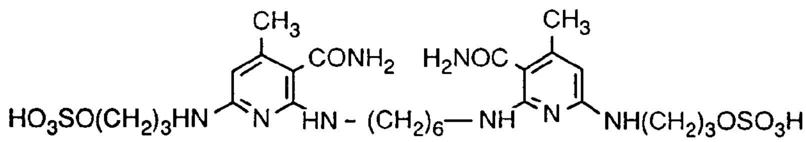

28

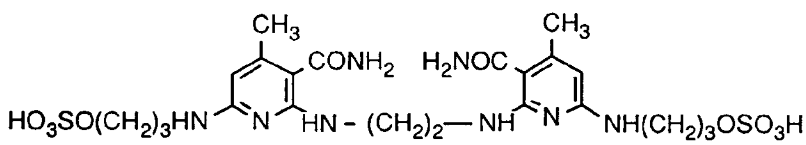

29

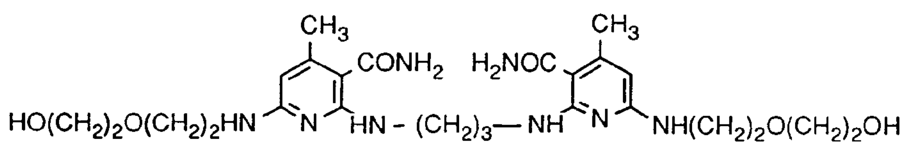

30

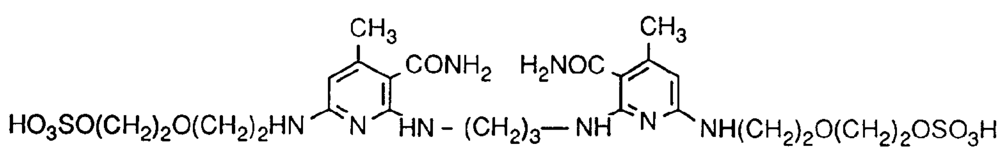

31

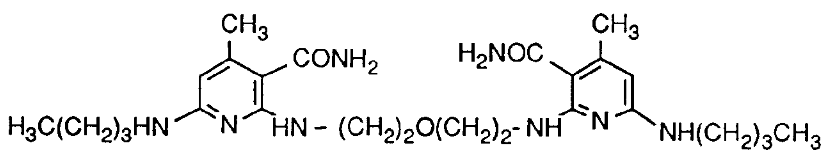

32 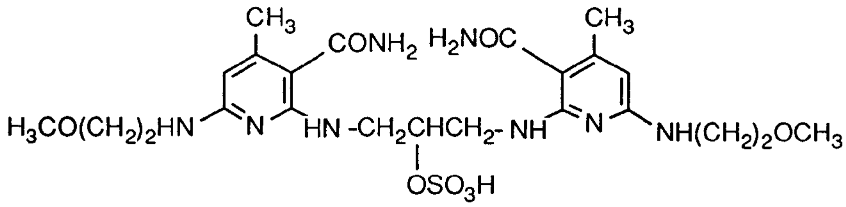

33 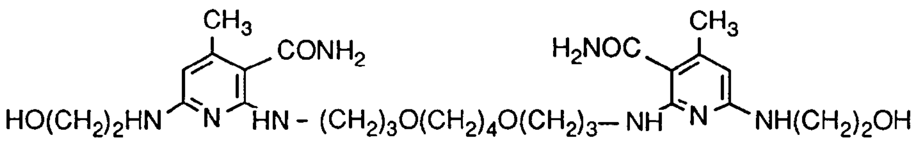

34 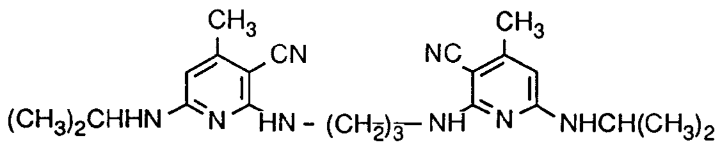

35 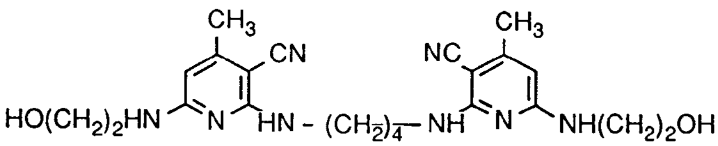

36 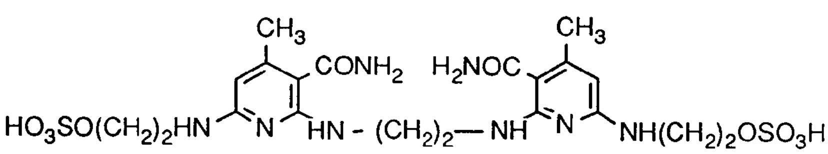

37 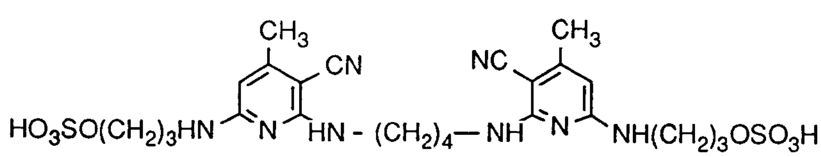

38

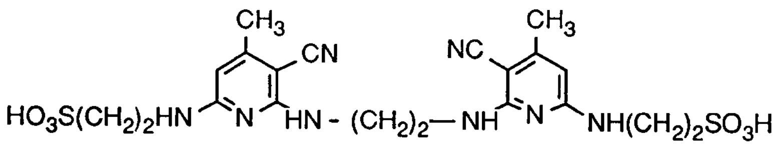

39

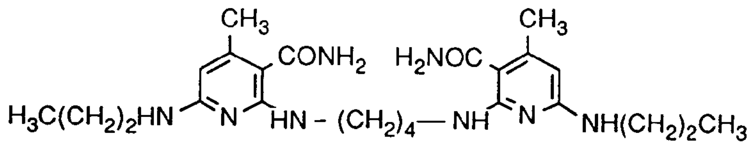

40

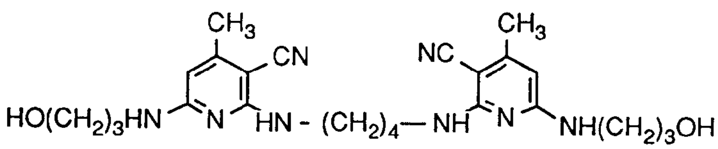

41

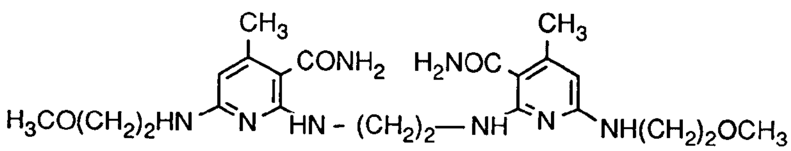

42

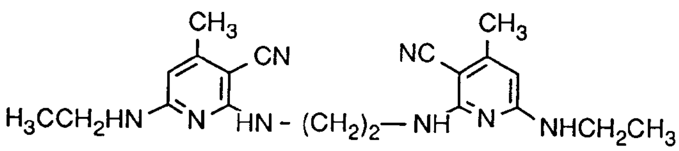

43

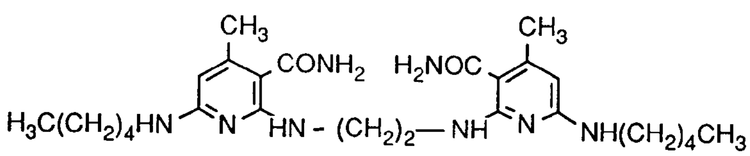

44

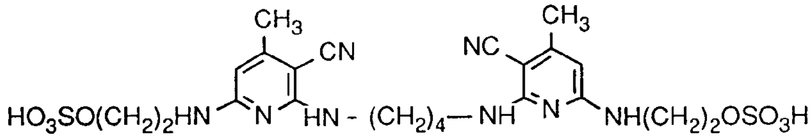

45

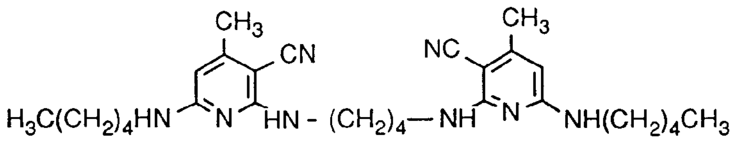

Farbstoff-Herstellungsbeispiel:

24,4 Teile Anilin-2-β-sulfatoäthylsulfon-S-sulfonsäure in 200 Teilen Eiswassersuspension werden mit 15 Teilen konzentrierter wässriger Salzsäure angesäuert und mit 13,6 Teilen einer 5-normalen Natriumnitritlösung diazotiert. Man rührt eine Stunde bei einer Temperatur von ca. 5°C nach und zerstört dann überschüssige salpetrige Säure mit Amidosulfonsäure. Die so hergestellte Diazoniumsalzlösung lässt man dann bei einem pH-Wert von 4 bis 5 langsam in eine Suspension einlaufen, welche in 100 Teilen Wasser 8 Teile eines Gemisches der Verbindungen der Formeln

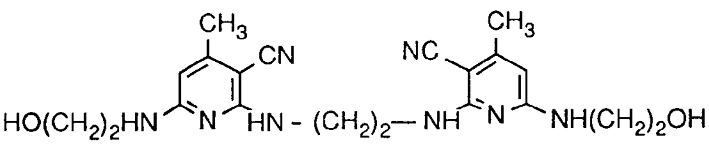

(112),

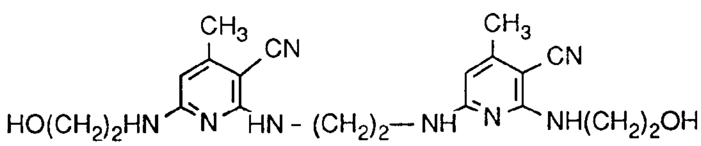

(113)

und

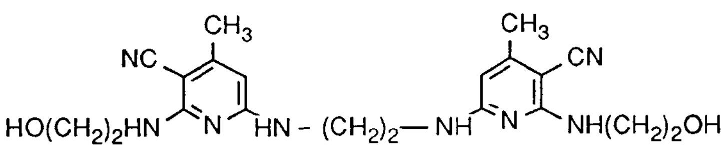

(114)

enthält, wobei das Verhältnis der Verbindungen der Formeln (112), (113) und (114) ca. 12:7:1 ist. Der pH-Wert wird durch Zugabe einer Natriumcarbonatlösung auf 6 bis 7 eingestellt. Man rührt 2 Stunden bis zur vollständigen Kupplung nach. Dann wird das erhaltene Produkt umkehrosmotisiert und gefriergetrocknet. Man erhält eine Mischung, welche die in Form der freien Säuren angegebenen Farbstoffe der Formeln

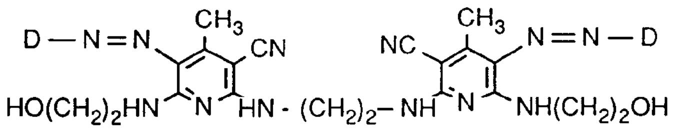

(115),

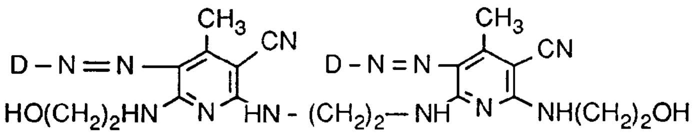

(116)

und

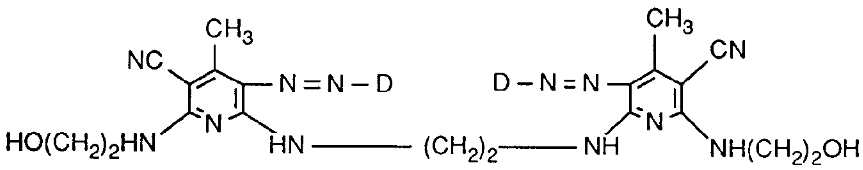

(117)

enthält, wobei in den Verbindungen der Formeln (115), (116) und (117) der Rest D jeweils ein Rest der Formel

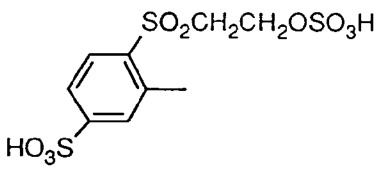

ist und das Verhältnis der Verbindungen der Formeln (115), (116) und (117) ca. 12:7:1 ist.

Die Mischung der Farbstoffe der Formeln (115), (116) und (117) färbt Baumwolle und Wolle in brillanten orangen Tönen.

**Patentansprüche**

1. Mischungen von Aminopyridinverbindungen, dadurch gekennzeichnet, dass sie eine Verbindung der Formel

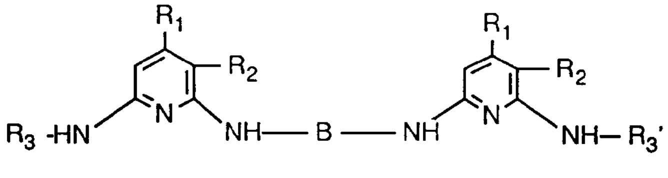

(1a)

zusammen mit mindestens einer Verbindung der Formeln

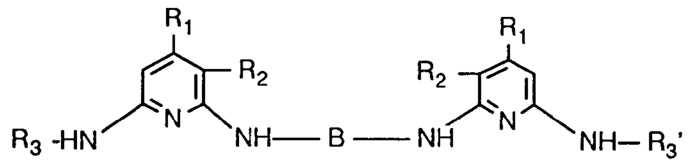

(1b)

und

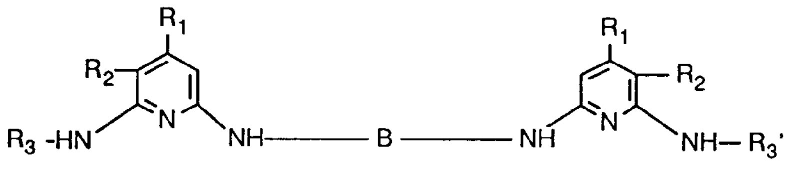

(1c)

enthalten, wobei
$R_1$ $C_1$-$C_4$-Alkyl,
$R_2$ Cyano, Carbamoyl oder Sulfomethyl ist,
$R_3$ und $R_3$' unabhängig voneinander Wasserstoff oder gegebenenfalls substituiertes und mit Ausnahme von Methyl gegebenenfalls durch Sauerstoff unterbrochenes $C_1$-$C_{12}$-Alkyl bedeuten und
B gegebenenfalls substituiertes und gegebenenfalls durch Sauerstoff unterbrochenes $C_2$-$C_{12}$-Alkylen ist.

2. Mischungen von Aminopyridinverbindungen gemäss Anspruch 1, dadurch gekennzeichnet, dass $R_1$ Methyl ist.

3. Mischungen von Aminopyridinverbindungen gemäss einem der Ansprüche 1 und 2, dadurch gekennzeichnet, dass $R_2$ Cyano oder Carbamoyl ist.

4. Mischungen von Aminopyridinverbindungen gemäss einem der Ansprüche 1 bis 3, dadurch gekennzeichnet, dass $R_3$ und $R_3$' unabhängig voneinander Wasserstoff oder gegebenenfalls durch Hydroxyl, Sulfo, Sulfato oder gegebenenfalls weitersubstituiertes Phenyl oder Naphthyl substituiertes und mit Ausnahme von Methyl gegebenenfalls durch Sauerstoff unterbrochenes $C_1$-$C_{12}$-Alkyl sind.

5. Mischungen von Aminopyridinverbindungen gemäss einem der Ansprüche 1 bis 4, dadurch gekennzeichnet, dass $R_3$ und $R_3$' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes $C_1$-$C_{12}$-Alkyl sind.

6. Mischungen von Aminopyridinverbindungen gemäss einem der Ansprüche 1 bis 5, dadurch gekennzeichnet, dass B gegebenenfalls durch Hydroxyl, Sulfo oder Sulfato substituiertes und gegebenenfalls durch Sauerstoff unterbrochenes $C_2$-$C_{12}$-Alkylen ist.

7. Mischungen von Aminopyridinverbindungen gemäss einem der Ansprüche 1 bis 6, dadurch gekennzeichnet, dass B $C_2$-$C_6$-Alkylen bedeutet.

8. Mischungen von Aminopyridinverbindungen gemäss einem der Ansprüche 1 bis 7, dadurch gekennzeichnet, dass $R_3$ und $R_3$' identische Bedeutungen haben.

9. Mischungen von Aminopyridinverbindungen gemäss einem der Anspüche 1 bis 8, dadurch gekennzeichnet, dass $R_1$ Methyl, $R_2$ Cyano oder Carbamoyl ist, $R_3$ und $R_3$' identische Bedeutungen haben und Wasserstoff oder gegebenenfalls durch Hydroxyl, Sulfo oder Sulfato substituiertes und mit Ausnahme von Methyl gegebenenfalls durch Sauerstoff unterbrochenes $C_1$-$C_{12}$-Alkyl sind und B gegebenenfalls durch Hydroxyl, Sulfo oder Sulfato substituiertes und gegebenenfalls durch Sauerstoff unterbrochenes $C_2$-$C_{12}$-Alkylen ist.

**10.** Verfahren zur Herstellung von Mischungen von Aminopyridinverbindungen gemäss Anspruch 1, dadurch gekennzeichnet, dass man eine Verbindung der Formel

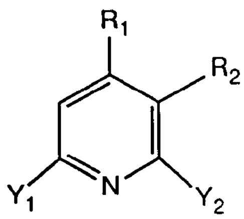

(2),

worin $R_1$ und $R_2$ die in Anspruch 1 angegebenen Bedeutungen haben und $Y_1$ und $Y_2$ je eine Abgangsgruppe sind,
mit Aminen der Formeln

$NH_2$-$R_4$ (3) und $NH_2$-B-$NH_2$ (4),

worin B die in Anspruch 1 angegebenen Bedeutungen hat und $R_4$ die für $R_3$ und $R_3$' angegebenen Bedeutungen hat, umsetzt und gegebenenfalls eine weitere Umwandlungsreaktion anschliesst.

**11.** Verwendung der Mischungen von Aminopyridinverbindungen gemäss einem der Ansprüche 1 bis 9 bzw. der gemäss Anspruch 10 hergestellten Mischungen als Kupplungskomponenten für die Herstellung von Farbstoffen.

Europäisches Patentamt

# EUROPÄISCHER RECHERCHENBERICHT

Nummer der Anmeldung

EP 93 81 0507

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-A-2 438 130 (BASF AG)<br>* Seiten 10-12, Beispiel 1 * | 1-11 | C07D213/74<br>C09B33/12 |
| X | DE-A-2 454 492 (BASF AG)<br>* Seiten 13-15, Beispiel 1 * | 1-9,11 | |
| A | CHEMICAL ABSTRACTS, vol. 100, no. 2, 9. Januar 1984, Columbus, Ohio, US;<br>abstract no. 8494t,<br>Seite 93 ;<br>& JP-A-58 101 158 (SUMITOMO CHEM. CO., LTD.)<br>16. Juni 1983 | 1-11 | |

| RECHERCHIERTE SACHGEBIETE (Int. Cl.5 ) |
|---|
| C07D<br>C09B |

Der vorliegende Recherchenbericht wurde für alle Patentansprüche erstellt

| Recherchenort | Abschlußdatum der Recherche | Prüfer |
|---|---|---|
| BERLIN | 28 OKTOBER 1993 | HASS C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)